# EUROPEAN PATENT SPECIFICATION

(11) **EP 0 711 546 B1**
(45) Date of publication and mention of the grant of the patent: **03.01.2001**
(21) Application number: 94926248.9
(22) Date of filing: 07.09.1994
(51) Int. Cl.: A61K 9/00

(54) **OPHTHALMIC SOLUTION BASED ON DICLOFENAC AND TOBRAMYCINE AND ITS APPLICATIONS**
OPHTHALMISCHE LÖSUNG, DIE DICLOFENAC UND TOBRAMYCIN ENTHÄLT, UND IHRE VERWENDUNG
SOLUTION OPHTALMIQUE A BASE DE DICLOFENAC ET TOBRAMICINE, ET SES APPLICATIONS

(30) Priority: 17.05.1994 ES 9401078
(43) Date of publication of application: 15.05.1996
(73) Proprietor: ALCON CUSI, S.A., El Masnou (Barcelona) (ES)
(72) Inventor: LOPEZ CABRERA, Antonio, E-08018 Barcelona (ES); TORRELLA CABELLO, Gemma, E-08320 El Masnou (Barcelona) (ES); VALLET MAS, José Alberto, E-08022 Barcelona (ES); BERGAMINI, Michael Van Wie, E-08320 El Masnou (Barcelona) (ES)
(74) Representative: Ungria Lopez, Javier
(86) International application number: PCT/ES94/00084
(87) International publication number: WO 95/31179

(56) References cited:
- EP-A- 0 390 071

## Description

The present invention is comprised within the technical field of formulations intended for the treatment of ocular and otic inflammation processes which are presented together with infections.

Specifically, the present invention offers a formulation based on diclofenac and tobramycin for its ophthalmic and otic topical use.

The non-steroidal antiinflammatory drugs (AINE) were introduced in ophthalmic practice as an alternative to the corticosteroids. At present, it is considered that the efficacy of the available AINEs can be compared to the less potent corticoids with the advantage that they are to be found a priori, lacking some of the negative effects associated with the use of any corticoids, unfavourable influence in certain types of infections -viruses, fungii and tuberculosis- and an increase of the intraocular pressure.

The association of an antibiotic and an antiinflammatory drug in ophthalmic pharmacy has had a generally useful result in inflammations associated with infections of ocular front segments, specially in conjunctivitis. In fact, data exist on severe bacteriological etiology conjunctivities which shows that the association of a corticoid with an antibiotic was more effective than a single antibiotic in the inactivation of the disorder [(1) Leibowitz HM. Human Conjunctivities II. Treatment. Arch. Ophthalmol. 1976; 94:175 2-6]. In this sense, it seems logical to think that the association of a type of antiinflammatory drug lacking the potential negative effects which are characteristic of the corticoids (for example, a AINE) with an antibiotic, may serve as therapeutical alternative.

It has also been observed that the surgical procedures of front segment (for example, cataract operations) are frequently associated with a postoperative inflammation which is reduced if a non-steroidal antiinflammatory drug is administered immediatly prior to the operation and is prolonged during the postoperative period [(Othenin-Girard PH Association diclofenac-dexamethasone dans le traitement de l'inflammation postopératoire: étude prospective en double-insu Klin Mbl. Augenheilk 1992;200:362-66; (3) Shiow-Wen Liou. The effect of 0.1 % Indomethacin eyedrops on cataract surgery. J. Ocular Pharmacol. 1991; 7:7 7 -81; (4) Flach AJ. The effect of Ketorolac tromethamine in reducing postoperative inflammation:double-mask parallel comparison with Dexamethasone. Ann. Ophtalmol. 1989; 19:407-411]

A correlation has been described between the blefaroconjuntival microbial flora present in the preoperative cataract surgery and the isolated germs of postoperative endophthalmitis [Parke DW. Endophthalmitis. In: K.Tabbara Infections of the eye. Little Brown & Oc. 1st. Edition. 1986 Boston (U.S.A.)]. For this reason, there is an agreement between the authors to suitably sterilize the external ocular structures whilst there is a possibility of infection in the surgical wound. A possibility used in the general practice is the topical administration of antibiotics with the correct spectrum in the pre- and postoperative period. The combination of a non steroidal antiinflammatory and of an antibiotic allows the approach to both aspects (inflammation and the possibility of infection).

From the previous description, it becomes clear that the possibility of having available a combination of an antiinflammatory agent and an antibiotic may seem necessary from the therapeutical point of view. On consideration of steroidal antiinflammatory agents, formulations can be found in the international market which combine the said compounds with antibiotics in general and with tobramycin in particular [(6) Vademecum International (1993) . 34th Edition. Medicom; (7) physicians' Desk Reference for Ophthalmology (1994). 22nd Edition. Medical Economics Data]

The combination of a non-steroidal antiinflammatory agent with antibiotics is not as documented as in the previous case, nor has any speciality containing the said association been commercialized. The Fu and Lidgate European Patent Application EP-A-0390071 is to be noted, which claims a preserving system for ophthalmic solutions which allow the compatibility of non-steroidal antiinflammatory agents, among others, ketorolac tromethamol and diclofenac, with preservatives belonging to the quaternary ammonium group by means of a non-ionic surfactant, specifically a polyethoxylate octyl phenol and preferably OCTOXYNOL 40, in the presence of tobramycin. According to the inventors, the formulations proposed avoid the interaction established between the AINEs and the quaternary ammoniums. Similar proposals have been carried out for formulations which only present quaternary ammonium (EP-0306984-B1 and US-A-4829088). For some of these compounds, it has been established that the interaction is due to an ion-pair formation among the carboxyl group of the antiinflammatory agents and the quaternary ammonium group [(8) Dreijer-Van der Glas, S.M. and Bult. A. Incompatibility of indomethacin and benzalkonium in eye drops due to ion-pair formation. Pharmaceutisch Weekblad Scientific Edition 1987;9:29-32; (9) Ogawa, T and Ohara, K. and Shimizu, H. Effects of Pretreatment with mydriatics on intraocular penetration of 0.1 % Pranoprofen. Jpn. J. Ophthalmol. 1993; 37:47-55]

When intending to develop a diclofenac and tobramycin ophthalmic formulation, some of the examples described in Patent EP-A-0390071, have been reproduced. Consequently, when combining tobramycin (0.3%) with tromethamol ketorolac (0.5%) a clear and transparent solution has been obtained. However, when conducting the same experiment with sodium diclofenac (0.5%) it has been observed that it is impossible to obtain a clear and transparent solution, observing solid particles without dissolving. In both cases, the same process of elaboration has been used, the simple mixture of the components, such as is described in the example of EP-A-0390071. The preparation, under the same conditions of single solutions with tobramycin (0.3%) or with sodium diclofenac (0.5%) has permitted to observe, that in the former case, a clear and transparent solution was obtained, whilst in the latter case, this was not possible, and only a cloudy solution could be obtained. All the formulations have been studied with the same quantity (0.01%) of one of the quaternary ammoniums used in ophthalmology, benzalkonium chloride (BAC). Solid particles without dissolving have also been observed when preparing a formulation which includes 0.1% of sodium diclofenac, which is the concentration generally used in ophthalmology. The presence of particles in suspension may be due to the following reasons:
- The insolubility of the sodium diclofenac under the experimental conditions used.
- The formation of an interaction between some of the dissolution components, as for example, between the diclofenac and the tobramycin, since when elaborating the formulation which only includes the tobramycin as active principle, a clear and transparent solution is obtained.

Paying attention to the bibliographic data consulted [(10) Morimoto, Y., Hatanaka, T. and Sugibayashi, K. and Omiya, H. Prediction of Skin Permeability of Drugs: Comparison of Human and Hairless Rat Skin. J. Pharm. Pharmacol 1992; 44:634- 6 39; (11) Kriwet, K. and Müller-goymann, C. binary Diclofenac Diethylamine-water systems: Micelles, vesicle and lyotropic liquid crystals. Eur J.Pharm, Biopharm. 1993; 39 ( 6) :234-238] the first reason can be emphasized, since at the working pH (7.4 ± 0.4), the diclofenac, presents a greater solubility to the concentration used in the experiments.

With the object of confirming the second possibility, formulations have been prepared with 0.1% (concentration generally used in ophthalmology) of sodium diclofenac, 0.3% of tobramycin (concentration generally used in ophthalmology), 0.01% of BAC, 1.0% of Octoxynol 40 (maximum concentration claimed in EP-A-0390071) and adjusting the pH to 8.0 (maximum value specified in EP-A-0390071). Two additional formulations have been prepared in parallel, one exclusively with sodium diclofenac as active principle, and the other only with tobramycin as active principle. Samples of the three formulations have been placed at 4 and 22ºC in order to follow the evolution thereof under critical conditions, from the point of view of the appearance of precipitates (4ºC), though realistic from the point of view of the conditions under which a preparation may be found during the life time of a pharmaceutical product, and under normal shelf-life conditions (22ºC). In the case of the formulation with the two active principles, the appearance of precipitates has been observed in a time < than 41 days, whilst in the other two formulations it has not been so. No appearance of precipitates has been observed at environmental temperature in any case whatsoever.

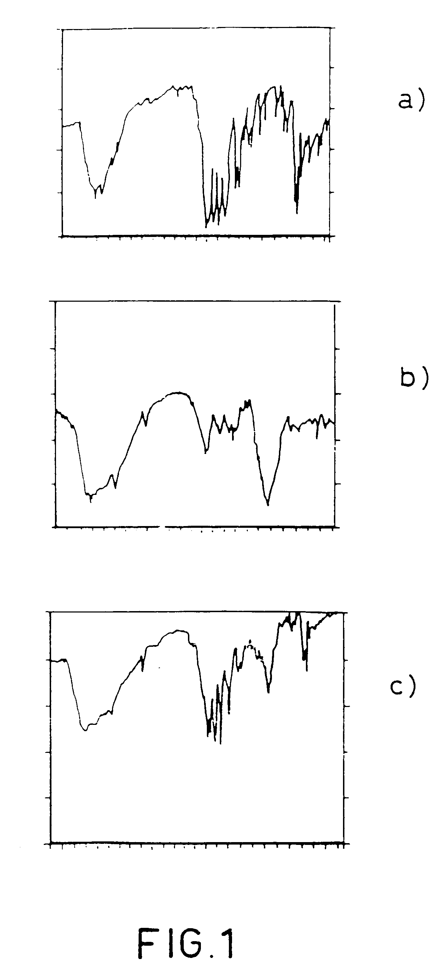

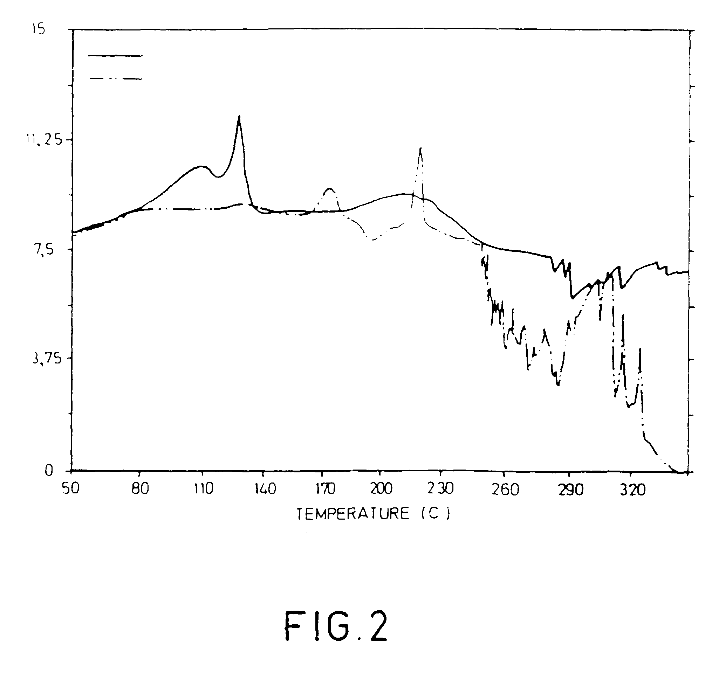

The precipitate formulated, was separated, and has been analyzed, with the detection in the same, of the presence of sodium diclofenac and tobramycin, both by thin layer chromatography and HPLC and by I.R. spectrography. The IR spectrum of the precipitate shows characteristic bands of each one of the active principles which are not found in the spectra of the individual components (see Figure 1). The DSC analysis of the precipitate shows a profile which is clearly differentiated from the one obtained with the individual components, as well as the profile obtained of the simple physical mixture of sodium diclofenac and tobramycin (see Figure 2).

The appearance of precipitation in a time < to 3 days has also been observed, even at 22ºC, with a formulation which contains 0.15% sodium diclofenac, 0.45% tobramycin, 1.0% OCTOXYNOL 40 and in which the pH has been adjusted to 8. These concentrations which are included in the claims of EP-A-0390071, were selected because it was considered that they are the maximums at which a formulation may be found with the normal concentrations of diclofenac and tobramycin in ophthalmology, 0.1% and 0.3% respectively, considering the need of having specifications for the elaboration of the formulations, the potential requirement of overdoses of the same, complying with the stability of the molecules [(6) Vademecum International (1993). 34th Edition. Medicom; (12) U.S.P. XXII (1990). United States Pharmacopeial Convention, INC:;(13) Brandl, M. and Gu, L. Degradation of Tobramycin in aqueous solution. Drug Development and Industrial Pharmacy, 1992; 18(3):1423-1436.] and to the concentration of the formulations by losses due to evaporation from the containers used generally for these products.

With the object of determining more widely, at which experimental conditions a clear and transparent solution cannot be obtained, a study has been conducted on the influence of the sodium diclofenac concentration, the OCTOXYNOL concentration and the pH in the combination of sodium diclofenac-tobramycin in the presence of BAC. As range for the sodium diclofenac concentrations, values comprised within 0.05 and 0.5% have been selected, values which include the values generally used in ophthalmology and which are included in the concentrations claimed in EP-A-0390071. As OCTOXYNOL concentrations, values comprised within 0.01 and 1.0% have been selected, this latter value being the one which corresponds with the maximum value claimed in EP-A-0390071. The pH studied have been 6, 7 and 8, values which coincide with the ones specified in EP-A-0390071. The concentration of tobramycin and BAC has been established in all cases at 0.3 and 0.01% respectively. The rest of the components have been set in all cases and corresponded to the examples specified in EP-A-0390071. The initial experimental plan has corresponded with a factorial design 3³ and as incidences have been observed, new formulations have been considered in order to limit the experimental conditions under which the formation of a clear and transparent solution containing sodium diclofenac and tobramycin may be observed. When proceeding with the elaboration of the different formulations and collection of the results, it was observed that the best conditions for obtaining clear and transparent solutions are those which correspond to the most alkaline pH from the ones studied and to the highest concentrations of surfactant. These facts manifest the contribution of the ion interaction, as well as with other AINEs and of hydrophobicals to the formation of the observed precipitate. It has been considered that with ph > 8, the tobramycin is to been found in the majority of cases in the non-ionic form which minimizes the electrostatic interaction.

After 30 days of storage in refrigerator, only clear and transparent solutions have been obtained under the following experimental conditions.

| pH | % OCTOXYNOL 40 | % Sodium Diclofenac |
|---|---|---|
| 6 | 1.0 | 0.05 |
| 7 | 1.0 | 0.05 |
| 8 | 0.5 | 0.05 |
| 8 | 1.0 | 0.05 |

After a minimum of 30 days storage at 22º C only clear and transparent solutions have been obtained under the following experimental conditions:

| pH | % OCTOXYNOL 40 | % Sodium Diclofenac |
|---|---|---|
| 6 | 1.0 | 0.05 |
| 7 | 1.0 | 0.05 |
| 7 | 0.5 | 0.05 |
| 8 | 0.25 | 0.05 |
| 8 | 0.5 | 0.05 |
| 8 | 1.0 | 0.05 |
| 8 | 1.0 | 0.10 |

The rest of the formulations studied have not offered clear and transparent solutions, either because they have not been obtained at zero time or because precipitations have appeared during the storage. It must also be pointed out that in the claims of EP-A-0390071, the pH is specified as 7.4 ± 0.4 for formulas covered by the same, and following the results obtained, it is observed that pH 7, and even at 22ºC and with the highest surfactant concentration claimed in EP-A-0390071, a clear and transparent solution cannot be obtained when using 0.1% sodium diclofenac and 0.3% tobramycin concentrations generally used in ophthalmology.

In general, the results obtained demonstrate a different behaviour between ketorolac and diclofenac, showing the existence of an interaction between sodium diclofenac and tobramycin. These differences could be explained by studying the special characteristics of diclofenac, such as has been referenced by various authors. In this way, an ion interaction could be established between tobramycin and sodium diclofenac, similar to the that established between the derivatives of quaternary ammonium and other non-steroidal antiinflammatory agents, and a hydrophobic interaction, also between the tobramycin and the sodium diclofenac, which is characteristic of this latter component. The ion interaction is probably established between the sodium diclofenac carboxyl group and the tobramycin amine groups, due to which, and studying the respective pKs, it is logical to think that the pH values used in the ophthalmic solutions, the pharmaceutically acceptable derivatives of the diclofenac and of the tobramycin also present the same interaction.

Experimental conditions are proposed in accordance with the present invention which permit the joint maintainance in solution of diclofenac and tobramycin in the concentrations generally used in ophthalmology, solving the problems detected when reproducing EP-A-0390071, when preventing the formation of interactions between diclofenac and tobramycin. Besides, the experimental conditions described allow the incorporation into the formulation, when it is believed necessary, of quaternary ammonium derivatives as preservatives, since the same also prevent the interaction between said compounds and diclofenac. The experimental conditions proposed allow to obtain solutions which are clear, transparent, stable, well tolerated and therapeutically efficient and which together contain diclofenac and tobramycin, preventing the limitations of the previously described prior art (EP-A-0390071, EP-0306984-B1 and US-A-4829088) which, since it does not consider the establishment of the interaction between said components, are not suitable to provide a formulation of diclofenac and tobramycin.

The present invention as claimed, refers to a solution of a non steroidal antiinflammatory agent, diclofenac, an antibiotic of the family of the aminoglycosides, tobramycin, and a solubilizing agent for its topical use in ocular and otic inflammatory processes which usually appear together with infection.

The ocular pathological processes which may be treated with the formulations described in the present invention are conjunctivitis or any other ocular trauma caused by an accident or a surgical operation. Additionally, the ocular inflammations and infections may also be treated with the formulations described in the present invention.

Likewise, with the formulations described in the present invention, diverse otic pathological processes may be treated, such as for example, external otitis. Moreover, the otic inflammations and infections may also be treated with the formulations described in the present invention.

Diclofenac is a non steroidal antiinflammatory agent which is chemically known as ortho(2,6-dichlorophenyl)aminophenylacetic acid. Diclofenac has the structural formula (I):

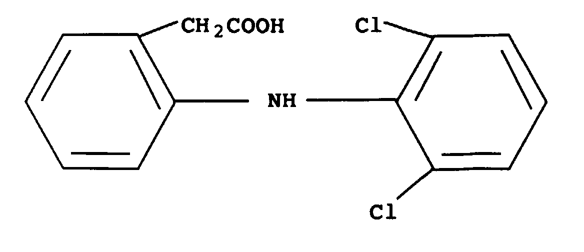

Tobramycin is an anti-bacterial agent belonging to the family of the aminoglycosides, water soluble, and chemically known as 4-[2,6-diamino-2,3,6-trideoxy-alpha-D-glycopyranosyl]-6-[3-amino-3-deoxy-alpha-D-glycopyranosyl]-2-deoxystreptamine. Tobramycin has the structural formula (II):

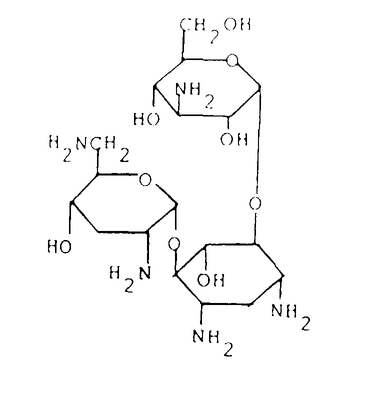

Tobramycin presents a wide spectrum of action, versus both Gram positive and versus Gram negative organisms. The main susceptible microorganisms are Staphylococus aureus, Staphylococus epidermidis, Streptococus pneumoniae, Pseudomonas aeruginosa, Escherichia coli, Enterobacter aerogenes, Proteus mirabilis, Klebsiella pneumoniae, Morganella morganii, Haemophilius influenzae, haemophilius aegyptius, Moraxela lacunata and Acinetobacter calcoaceticus.

Moreover, the present invention includes the isomers, derivatives and pharmaceutically acceptable salts of diclofenac and of tobramycin

In accordance with the present invention, the resultant combination includes the equivalence to a value comprised within 0.001 and 0.14%, obtained from diclofenac itself, or from a pharmaceutically acceptable derivative thereof, preferably the equivalence to a value between 0.05 and 0.10% obtained from diclofenac itself, or from a pharmaceutically acceptable derivative thereof, and the equivalence to a value between 0.001 and 0.45% of tobramycin obtained from tobramycin itself or from a pharmaceutically acceptable derivative thereof, preferably, the equivalence to a value between 0.15 and 0.35% of tobramycin obtained from tobramycin itself, or from a pharmaceutically acceptable derivative thereof.

The solutions included in the present invention, incorporate, as solubilizing agent, namely sorbitol esters, glycerolpolyethylene glycol fatty acid esters, or a mixture thereof. These compounds are used at levels between 3.0 and 7.0%.

The compositions comprised in the present invention also include other compounds traditionally used in ophthalmic preparations, such as tonicifier agents, pH buffering substances, viscosity agents, chelating agents and preservative agents.

The inclusion of any representative of the previously indicated compound groups depends on the characteristics which are required to be conferred to the final preparations. The selection of one or other compound depends on the physical, physico-chemical and chemical characteristics of the rest of the components in the formulation so as to obtain a stable, well tolerated and therapeutically efficient preparation.

In the following paragraphs, both in the description and claims, the percentages are expressed by weight/volume in those cases in which the resultant pharmaceutical form is a solution. When the resultant formulation is a hydrogel, the percentage is expressed by weight/weight.

As tonicifier agents, sodium chloride, sodium sulphate, glycol, mannitol and sorbitol, among others, may be cited. These compounds are used to achieve the tonics required in the preparations. Typically, these compounds are used at levels between 0.4 and 75%.

As pH buffering substances, citrates, borates, phosphates, tris- (hydroxymethyl)-aminomethane and aminoacids such as glycine and lysine, glutamic acid, arginine and aspartic acid, among others, may be included. This type of product is introduced in the formulations to maintain the pH stable during the life time of the product and improve the tolerance when thus required by the product's use. Typically, these compounds are used at levels between 0.01 and 3.0%

As viscosity agents which improve the time of residence of the product at its location of application, among others, polyvinyl alcohol, polyvinylpyrrolidone, methylcellulose, hydroxypropylmethylcellulose, may be mentioned. Typically these compounds are used at levels between 0.01 and 10.0%.

As chelating agents, among others, citric acid, ethylendiamine tetraacetic acid (EDTA), EDTA sodium salts and ethyleneglycol-bis(beta-aminoethylether)N,N-tetraacetic acid (EGTA). These compounds are included in order to eliminate heavy metals from the solution and to improve the performance of the preservative agents. Typically, these compounds are used at levels between 0.01 and 2.0%.

As preservative agents, in order to prevent the contamination of the product when the formulation is presented in a multidose format, derivatives of quaternary ammonium (benzalkonium chloride, cetylmethylammonium bromide, cetylpyridine chloride and benzethonium chloride), organomercurial derivatives (thimerosal, phenyl mercuric acetates and phenylmercuric nitrate), methyl and propyl p-hydroxybenzoates and the sodium salts thereof, beta-phenylethyl alcohol, benzyl alcohol, phenylethyl alcohol and phenoxyethanol. The formulations of the present invention may also include a mixture of said compounds. Typically, these compounds are used at levels between 0.0005 and 5.0%, depending on the type of preservative agents selected.

Other optional excipients which may be used depending on the final characteristics which are desired to be conferred to the preparation, may be excipients generally used for obtaining pharmaceutical hydrogels e.g. poly(hydroxyethylmethacrylate), poly(N-vinylpyrrolidone), polyvinyl alcohol, polymers of acrylic acid, such as Carbopol, etc.). Typically, these compounds are used at levels between 0.01 and 25.0%.

The characteristics of the selected components may condition the pharmaceutical form necessary for obtaining a stable, well tolerated and therapeutically efficient preparation.

The pH of the formulations included in the present invention is between 7 and 9. In order to adjust the pH of the formulations to the desired value, besides the previously indicated buffering agents, acids (hydrochloric, sulphuric, etc) or bases (sodium hydroxide, potassium hydroxide, etc) may be used.

The quantity of preparation which may be administered to the receptor animal depends on the nature of the latter (species, age, size) as well as the general health condition and the severity and type of illness it suffers. Though the schedule of the dose has been established by the doctor or veterinarian, it is recommended that the application of the formulations included in the present invention be carried out from 1 to 4 times a day, depending on the characteristics of the formulation, instilling one or two drops each time.

The formulations included in the present invention may be packaged in containers generally used for this type of preparation.

When required, the formulations of the present invention may be elaborated under sterile conditions.

The presence of tobramycin, or of a pharmaceutically acceptable derivative thereof, does not affect the activity of the antiinflammatory agent used, neither does the presence of diclofenac, or of a pharmaceutically acceptable derivative thereof, interfere in the antimicrobial activity of the antibiotic.

Using an adequate combination of the components described in the present invention, formulations are obtained with effective results, from the antimicrobial point of view, according to the criteria of the different pharmacopoeias.

Figure 1 (a) IR spectrum of sodium diclofenac (b), IR spectrum of tobramycin. (c) IR spectrum of the precipitate obtained from a formulation with 0.1% sodium diclofenac, 0.3% tobramycin, 0.01% BAC, 1.0% OCTOXYNOL 40 and pH 8.0. Bands are observed at approximately 1.500 cm⁻¹, characteristic of the sodium diclofenac carboxyl groups, and a band, at approximately 1.050 cm⁻¹, characteristic of the tobramycin C-N and C-O groups.

Figure 2. DSC profile, obtained with the physical mixture (M) of sodium diclofenac and tobramycin and with precipitate (P) obtained from a formulation with 0.1% sodium diclofenac, 0.3% tobramycin, 0.01% BAC, 1.0% OCTOXYNOL 40 and pH 8.0.

The present invention is additionally represented by means of the following non-limitative examples.

### EXAMPLE NO.1

| Substance | Quantity for 100ml |
|---|---|
| Sodium diclofenac | 0.100 g |
| Tobramycin | 0.300 g |
| Benzalkonium chloride | 0.010 g |
| Polysorbate 80 | 3.000 g |
| Boric acid | 0.900 g |
| Sodium tetraborate | 0.450 g |
| EDTA Na₂ | 0.100 g |
| NaCl c.s.p | 300 m 0 s mol/ Kg |
| HCl and/or NaOH c.s.p. | pH 8.4 0.4 |
| Pure water x.s.p | 100 ml |

For obtaining the ophthalmic solution, 80% water of the formulation is placed in an adequate container. The benzalkonium chloride, the boric acid, the tetraborate, the EDTA Na₂, the NaCl, the Polysorbate 80, the tobramycin and the sodium diclofenac are added. The pH is adjusted with HC1 and/or NaOH, and the volume is completed with water. The resulting solution is filtered through a previously sterilized filtration system of 0.22 micron. The dose of the solution obtained is measured into adequate, previously sterilized flasks.

### EXAMPLE NO.2

| Substance | Quantity for 100 ml |
|---|---|
| Sodium diclofenac | 0.100 g |
| Tobramycin | 0.300 g |
| Benzalkonium chloride | 0.010 g |
| Polysorbate 20 | 3.000 g |
| Boric acid | 0.900 g |
| Sodium tetraborate | 0.450 g |
| EDTA Na₂ | 0.100 g |
| NaCl c.s.p. | 300 mosmol/Kg |
| HCl and/or NaOH c.s.p | pH 8.4 .4 |
| Pure water c.s.p | 100 ml |

For obtaining the ophthalmic solution, 80% of the water of the formulation is put into an adequate container. The benzalkonium chloride, the boric acid, the tetraborate, the EDTA Na₂, the NaCl, the polysorbate 20, the tobramycin and the sodium diclofenac are added. The pH is adjusted with HCl and /or NaOH, and the volume is completed with water and the resultant solution is filtered through a previously sterilized filtration system of 0.22 micron. The dose of the solution obtained is measured into adequate, previously sterilized flasks.

### EXAMPLE NO.3

| Substance | Quantity for 100 ml |
|---|---|
| Sodium diclofenac | 0.100 g |
| Tobramycin | 0.300 g |
| Benzalkonium chloride | 0.010 g |
| Glycerolpolyethylene glycol Ricinoleate | 3.500 g |
| Tromethamol | 0.600 g |
| EDTA Na₂ | 0.100 g |
| NaCl c.s.p. | 300 m0smol/Kg |
| H₂SO₄ and/or NaOH c.s.p | pH 8.4 ± 0.4 |
| Pure water c.s.p | 100 ml |

For obtaining the ophthalmic solution, 80% of the water of the formulation are put into an adequate container. The benzalkonium chloride, the tromethamol, the EDTA Na₂, the NaCl, the glycerolpolyethylene glycol ricinoleate, the tobramycin and the sodium diclofenac are added. The pH is adjusted with H₂SO₄ and /or NaOH, and the volume is completed with water and the resulting solution is filtered through a previously sterilized filtration system of 0.22 micron. The dose of the solution obtained is measured into adequate, previously sterilized flasks.

### EXAMPLE NO.4

| Substance | Quantity for 100 ml |
|---|---|
| Sodium Diclofenac | 0.100 g |
| Tobramycin | 0.300 g |
| Benzalkonium Chloride | 0.010 g |
| Polysorbate 80 | 3.000 g |
| Hydroxypropylmethylcellulose | 0.300 g |
| Boric acid | 0.900 g |
| Sodium tetraborate | 0.450 g |
| EDTA Na₂ | 0.100 g |
| NaCl c.s.p. | 300 m0smol/ Kg |
| HCl and/or NaOH c.s.p. | pH 8.4±0.4 |
| Pure water c.s.p | 100 ml |

For obtaining the ophthalmic solution, 80% of the water of the formulation are put into an adequate container. The benzalkonium chloride, the hydroxypropylmethylcellulose, the boric acid, the tetraborate, the EDTA Na₂, the NaCl, the Polysorbate 80, the tobramycin and the sodium diclofenac are added. The pH is adjusted with HC1 and/or NaOH, and the volume is completed with water and the resultant solution is filtered through a previously sterilized filtration system of 0.22 micron. The dose of the solution obtained, is measured in adequate, previously sterilized flasks.

### EXAMPLE NO. 5

| Substance | Quantity for 100 ml |
|---|---|
| Sodium diclofenac | 0.100 g |
| Tobramycin | 0.300 g |
| Benzalkonium chloride | 0.010 g |
| Polysorbate 20 | 3.000 g |
| Hydroxyethylcellulose | 0.500 g |
| Boric acid | 0.900 g |
| Sodium tetraborate | 0.450 g |
| EDTA Na₂ | 0.100 g |
| NaCl c.s.p. | 300 m0smol/Kg |
| HCl and/or NaOH c.s.p. | pH 8.0±0.4 |
| Pure water c.s.p | 100 ml |

For obtaining the ophthalmic solution, 80% of the water of the formulation are put into an adequate container. The benzalkonium chloride, the hydroxyethylcellulose, the boric acid, the tetraborate, the EDTA Na₂, the NaCl, the Polysorbate 20, the tobramycin and the sodium diclofenac are added. The pH is adjusted with HC1 and/or NaOH, and the volume is completed with water and the resulting solution is filtered through a previously sterilized filtration system of 0.22 micron. The dose of the solution obtained, is measured into adequate, previously sterilized flasks.

## Claims

1. An ophthalmic solution based on diclofenac and tobramycin for topical ophthalmic and otic use, characterized in that it has a pH of from 7 to 9 and in that it comprises, by weight/volume when formulated as a solution as such, or by weight/weight when formulated as a hydrogel,
- the equivalent to a value between 0.001-14% diclofenac, obtained from diclofenac itself, or from an isomer, or from a derivative or one of the pharmaceutically acceptable salts thereof;
- the equivalent to a value between 0.001-0.45% of tobramycin, obtained from tobramycin itself, or from an isomer, or from a derivative or one of the pharmaceutically acceptable salts thereof;
- 3.0 - 7.0% of a solubilizing agent selected from the group consisting of sorbitol esters, glycerolpolyethylene glycol fatty acid esters, and mixtures thereof; and
- optionally, 0.4-7.5% of a tonicifier;
- optionally, 0.01-3.0% of a pH buffering agent;
- optionally, 0.01-10.0% of a viscosity agent;
- optionally, 0.01-2.0% of a chelating agent;
- optionally,0.0005-0.015% of a preservative agent of the quaternary ammonium group;
- optionally 0.0005-0.015% of a preservative agent of the mercurial derivatives group;
- optionally, 0.05-0.5% of a preservative of the methyl and propyl p-hydroxybenzoate group and the sodium salts thereof;
- optionally, 0.05-0.75% of phenylethyl alcohol or beta-phenylethyl alcohol as preservative agent;
- optionally, 0.05-5.0% of benzyl alcohol as preservative agent;
- optionally, 0.05-1.0% of phenoxyethanol as preservative agent;
- optionally, 0.05-1.0% of phenoxyethanol as preservative agent;
- optionally, 0.01-25% of an excipient used for pharmaceutical hydrogels.

2. A solution according to claim 1, characterized in that it comprises 0.05-0.1% of diclofenac.

3. A solution according to claim 1, characterized in that it comprises 0.15-0.35% of tobramycin.

4. A solution according to claim 1, characterized in that the tonicifier is selected from the group formed by sodium chloride, sodium sulphate, glycerol, mannitol and sorbitol.

5. A solution according to claim 1, characterized in that the buffering agent is selected from the group formed by acetates, citrates, borates, phosphates, tris(hydroxymethyl)-aminoethane, and aminoacids such as glycine, lysine, glutamic acid, arginine and aspartic acid.

6. A solution according to claim 1, characterized in that the viscosity agent is selected from the group formed by polyvinyl alcohol, polyvinylpyrrolidone, methylcellulose, hydroxypropylcellulose, hydroxyethylcellulose, carboxymethylcellulose and hydroxypropylmethylcellulose.

7. A solution according to claim 1, characterized in that the chelating agent is selected from the group formed by citric acid, ethylenediaminetetraacetic acid (EDTA), EDTA sodium salts and ethyleneglycol-bis(beta-aminoethylether)N,N-tetraacetic acid (EGTA).

8. A solution according to claim 1, characterized in that the preservative agent is selected from the group formed by quaternary ammonium derivatives (benzalkonium chloride, cetylmethylammonium bromide, cetylpyridine chloride and benzethonium chloride), organomercurial derivatives (thimerosal, phenylmercuric acetate and phenylmercuric nitrate), or a mixture thereof and of the other preservative agents defined as optional in claim 1.

9. A solution according to claim 1, characterized in that the excipient for the pharmaceutical hydrogels is selected from the group formed by poly(hydroxyethylmethacrylate), poly(N-vinylpyrrolidone), polyvinyl alcohol and acrylic acid polymers, such as Carbopol.

10. Use of a formulation of any of claims 1 to 9 for the manufacture of medicines for the treatment of ocular inflammations and/or infections.

11. Use of a formulation of any of claims 1 to 9 for the manufacture of medicines for the treatment of otic inflammations and/or infections.

## Patentansprüche

1. Ophthalmische Lösung basierend auf Diclofenac und Tobramycin für eine örtliche ophthalmische und otische Verwendung, **dadurch gekennzeichnet,** dass sie einen pH von 7 bis 9 aufweist und dass sie, bezogen auf Gewich/Volumen, wenn sie als Lösung als solche formuliert wird, oder bezogen auf Gewicht/Gewicht, wenn sie als Hydrogel formuliert wird, umfasst:
- das Äquivalent zu einem Wert zwischen 0,001-14 % Diclofenac, erhalten von Diclofenac selbst oder von einem Isomer oder von einem Derivat oder einem der pharmazeutisch annehmbaren Salze davon;
- das Äquivalent zu einem Wert zwischen 0,001-0,45 % Tobramycin, erhalten von Tobramycin selbst oder von einem Isomer oder von einem Derivat oder einem der pharmazeutisch annehmbaren Salze davon;
- 3,0 - 7,0 % eines löslich machenden Mittels, ausgewählt aus der Gruppe bestehend aus Sorbitolestem, Glycerolpolyethylenglycol-Fettsäureestem und Gemischen davon; und
- optional 0,4-7,5 % eines Tonizitätsmittels (tonicifier);
- optional 0,01-3,0 % einer pH-Puffersubstanz;
- optional 0,01-10,0 % eines Viskositätsmittels;
- optional 0,01-2,0 % eines Chelatbildners;
- optional 0,0005-0,015 % eines Konservierungsmittels aus der Gruppe der quatemären Ammonium-Verbindungen;
- optional 0,0005-0,015 % eines Konservierungsmittels aus der Gruppe der Quecksilberderivate;
- optional 0,05-0,5 % eines Konservierungsmittels aus der Gruppe von Methyl- und Propyl-p-hydroxybenzoat und der Natriumsalze davon;
- optional 0,05-0,75 % Phenylethylalkohol oder Beta-phenylethylalkohol als Konservierungsmittel;
- optional 0,05-5,0 % Benzylalkohol als Konservierungsmittel;
- optional 0,05-1,0 % Phenoxyethanol als Konservierungsmittel;
- optional 0,01-25 % eines Exzipiens, das für pharmazeutische Hydrogele verwendet wird.

2. Lösung nach Anspruch 1, **dadurch gekennzeichnet,** dass sie 0,05-0,1 % Diclofenac umfasst.

3. Lösung nach Anspruch 1, **dadurch gekennzeichnet,** dass sie 0,15-0,35 % Tobramycin umfasst.

4. Lösung nach Anspruch 1, **dadurch gekennzeichnet,** dass das Tonizitätsmittel aus der durch Natriumchlorid, Natriumsulfat, Glycerol, Mannitol und Sorbitol gebildeten Gruppe ausgewählt ist.

5. Lösung nach Anspruch 1, **dadurch gekennzeichnet,** dass die Puffersubstanz aus der durch Acetate, Citrate, Borate, Phosphate, Tris(hydroxymethyl)aminoethan und Aminosäuren wie Glycin, Lysin, Glutaminsäure, Arginin und Asparaginsäure gebildeten Gruppe ausgewählt ist.

6. Lösung nach Anspruch 1, **dadurch gekennzeichnet,** dass das Viskositätsmittel aus der durch Polyvinylalkohol, Polyvinylpyrrolidon, Methylcellulose, Hydroxypropylcellulose, Hydroxyethylcellulose, Carboxymethylcellulose und Hydroxypropylmethylcellulose gebildeten Gruppe ausgewählt ist.

7. Lösung nach Anspruch 1, **dadurch gekennzeichnet,** dass der Chelatbildner aus der durch Citronensäure, Ethylendiamintetraessigsäure (EDTA), EDTA-Natriumsalze und Ethylenglycol-bis(beta-aminoethylether)N,N-tetraessigsäure (EGTA) gebildeten Gruppe ausgewählt ist.

8. Lösung nach Anspruch 1, **dadurch gekennzeichnet,** dass das Konservierungsmittel aus der durch quaternäre Ammoniumderivate (Benzalkoniumchlorid, Cetylmethylammoniumbromid, Cetylpyridinchlorid und Benzethoniumchlorid), Organoquecksilberderivate (Thimerosal, Phenylquecksilberacetat und Phenylquecksilbernitrat) oder einer Mischung davon und aus den anderen Konservierungsmitteln, die in Anspruch 1 als optional definiert sind, gebildeten Gruppe ausgewählt ist.

9. Lösung nach Anspruch 1, **dadurch gekennzeichnet,** dass das Exzipiens für die pharmazeutischen Hydrogele aus der durch Poly(hydroxyethylmethacrylat), Poly(N-vinylpyrrolidon), Polyvinylalkohol und Acrylsäurepolymere, wie etwa Carbopol, gebildeten Gruppe ausgewählt ist.

10. Verwendung einer Formulierung nach einem der Ansprüche 1 bis 9 zur Herstellung von Arzneimitteln für die Behandlung von Augenentzündungen und/oder -infektionen.

11. Verwendung einer Formulierung nach einem der Ansprüche 1 bis 9 für die Herstellung von Arzneimitteln für die Behandlung von otischen Entzündungen und/oder Infektionen.

## Revendications

1. Solution ophtalmique à base de diclofénac et de tobramycine pour un usage topique ophtalmique et auriculaire, caractérisée en ce qu'elle a un pH de 7 à 9 et en ce qu'elle comprend, en masse sur volume quand elle est formulée sous la forme d'une solution telle quelle, ou en masse/masse quand elle est formulée sous la forme d'un hydrogel,
- l'équivalent d'une valeur entre 0,001 et 14 % de diclofénac obtenu à partir du diclofénac lui-même ou d'un de ses isomères, ou d'un de ses dérivés ou d'un de leurs sels pharmaceutiquement acceptables ;
- l'équivalent d'une valeur entre 0,001 et 0,45 % de tobramycine obtenu à partir de la tobramycine elle-même ou d'un de ses isomères, ou d'un de ses dérivés ou d'un de leurs sels pharmaceutiquement acceptables ;
- 3,0 - 7,0 % d'un agent solubilisant choisi dans le groupe constitué par les esters de sorbitol, les esters d'acides gras du glycérolpolyéthylèneglycol et leurs mélanges ; et
- éventuellement, 0,4 - 7,5 % d'un agent d'ajustement de la pression osmotique ;
- éventuellement, 0,01 - 3,0 % d'un agent tampon pour l'ajustement du pH ;
- éventuellement, 0,01 - 10,0 % d'un agent d'ajustement de la viscosité ;
- éventuellement, 0,01 - 2,0 % d'un agent chélatant ;
- éventuellement, 0,0005 - 0,015 % d'un agent conservateur du groupe des ammoniums quaternaires ;
- éventuellement, 0,0005 - 0,015 % d'un agent conservateur du groupe des dérivés mercuriels ;
- éventuellement, 0,05 - 0,5 % d'un agent conservateur du groupe des p-hydroxybenzoates de méthyle et de propyle et de leurs sels de sodium ;
- éventuellement, 0,05 - 0,75 % d'alcool phényléthylique ou d'alcool béta-phényléthylique en tant qu'agent conservateur ;
- éventuellement, 0,05 - 5,0 % d'alcool benzylique en tant qu'agent conservateur ;
- éventuellement, 0,05 - 1,0 % de phénoxyéthanol en tant qu'agent conservateur ;
- éventuellement, 0,01 - 25 % d'un excipient utilisé pour les hydrogels pharmaceutiques.

2. Solution selon la revendication 1, caractérisée en ce qu'elle comprend 0,05 - 0,1 % de diclofénac.

3. Solution selon la revendication 1, caractérisée en ce qu'elle contient 0,15 - 0,35 % de tobramycine.

4. Solution selon la revendication 1, caractérisée en ce que l'agent pour l'ajustement de la pression osmotique est choisi dans le groupe constitué par le chlorure de sodium, le sulfate de sodium, le glycérol, le mannitol et le sorbitol.

5. Solution selon la revendication 1, caractérisée en ce que l'agent tampon est choisi dans le groupe constitué par les acétates, les citrates, les borates, les phosphates, le tris(hydroxyméthyl)aminoéthane et les aminoacides tels que la glycine, la lysine, l'acide glutamique, l'arginine et l'acide aspartique.

6. Solution selon la revendication 1, caractérisée en ce que l'agent pour l'ajustement de la viscosité est choisi dans le groupe constitué par l'alcool polyvinylique, la polyvinylpyrrolidone, la méthylcellulose, l'hydroxypropylcellulose, l'hydroxyéthylcellulose, la carboxyméthylcellulose et l'hydroxypropylméthylcellulose.

7. Solution selon la revendication 1, caractérisée en ce que l'agent chélatant est choisi dans le groupe constitué par l'acide citrique, l'acide éthylènediaminetétraacétique (EDTA), les sels de sodium de l'EDTA et l'acide éthylèneglycol-bis(béta-aminoéthyléther)-N,N-tétraacétique (EGTA).

8. Solution selon la revendication 1, caractérisée en ce que l'agent conservateur est choisi dans le groupe constitué par les dérivés d'ammoniums quaternaires (chlorure de benzalconium, bromure de cétylméthylammonium, chlorure de cétylpyridine et chlorure de benzéthonium), les dérivés organomercuriels (thimérosal, acétate phénylmercurique et nitrate phénylmercurique) ou un de leurs mélanges et les autres agents conservateurs définis comme facultatifs dans la revendication 1.

9. Solution selon la revendication 1, caractérisée en ce que l'excipient pour les hydrogels pharmaceutiques est choisi dans le groupe constitué par le polyméthacrylate d'hydroxyéthyle, la poly(N-vinylpyrrolidone), l'alcool polyvinylique et les polymères de l'acide acrylique tels que le Carbopol.

10. Utilisation d'une formulation selon une quelconque des revendications 1 à 9 pour la fabrication de médicaments pour le traitement d'inflammations et/ou d'infections oculaires.

11. Utilisation d'une formulation selon une quelconque des revendications 1 à 9 pour la fabrication de médicaments pour le traitement d'inflammations et/ou d'infections auriculaires.
